# EUROPEAN PATENT APPLICATION

(11) **EP 4 390 842 A1**
(43) Date of publication of application: **26.06.2024**
(21) Application number: 22215997.2
(22) Date of filing: 22.12.2022
(51) Int. Cl.: G06T 7/11

(54) **DEEP-LEARNING BASED INTERACTIVE SEGMENTATION FOR MEDICAL VOLUMETRIC IMAGING DATASETS**

(71) Applicant: Siemens Healthineers AG, 91301 Forchheim (DE)
(72) Inventor: Jendryka, Julian, 96052 Bamberg (DE); Dr. Katzmann, Alexander, 90765 Fürth (DE); Thamm, Florian, 90762 Fürth (DE); Dr. Sühling, Michael, 91052 Erlangen (DE)
(74) Representative: Siemens Healthineers Patent Attorneys

(57) **Abstract**

The disclosure relates to an interactive segmentation process based on a neural network algorithm. Corrective or ascertaining user input is encoded and input to the neural network algorithm.

## Description

Various examples of the disclosure generally pertain to segmentation of imaging datasets. Various examples specifically pertain to an interactive segmentation process for volumetric imaging datasets.

The segmentation of organs and other regions of interest plays a crucial role in diagnostic imaging. The purpose of such segmentation is manifold. It can either directly be used for visualization purposes, such as typically conducted for vessels, or as a surrogate or intermediate result for algorithms analyzing said region.

In clinical practice, both texture and shape of segmented regions are important factors for the assessment in diverse fields, such as oncology and oncologic treatment response analysis (cf. radiomics), cardiovascular analysis and cardiac risk assessment (cf. CAD-RADS), computed tomography angiography (CTA), and many others.

There is a need for segmentation processes to segment medical imaging data, specifically volumetric imaging datasets.

This need is met by the features of the independent claims. The features of the dependent claims define embodiments.

Independent of the grammatical term usage, individuals with male, female or other gender identities are included within the term.

Hereinafter, interactive segmentation procedures will be disclosed. The interactive segmentation processes employ a neural network algorithm (NN) to determine, for each iteration, a current estimate of the segmentation. Based on user feedback, this current estimate is then refined in the next iteration (hence, the process is interactive). Thus, an a-posterior estimate of the segmentation of a given iteration serves as the a-priori estimate of the segmentation in the subsequent iteration.

The segmentation can be represented by a binary mask, delimitating parts or regions included in the segmented region of interest (ROI) from parts or regions not included in the segmented ROI.

According to some examples, the NN can obtain, as an input, a volumetric imaging dataset. This corresponds to processing three-dimensional (3-D) data. 3-D data may be obtained, e.g., from a CT apparatus or a Magnetic Resonance Imaging (MRI) apparatus. According to other examples, the NN can obtain a 2-D image. 2-D images may be slices of a 3-D dataset. For 3-D data, inputs to the NN can be encoded as 3-D array data structures, i.e., cuboid data structures. Each array entry corresponds to a certain position in 3-D space (xyz-position). For 2-D data, inputs can be encoded as 2-D array data structures (xy-position) .

The 3-D segmentation can be represented by a 3-D array data structure; the 2-D segmentation by a 2-D array data structure.

According to some examples, the NN can obtain as a further input, a history of user inputs that correct or ascertain the respective estimates of the segmentation for multiple iterations of the interactive segmentation process. By considering user inputs not only for the last iteration, but multiple iterations, more accurate refinement is possible.

Patch-by-patch processing of inputs provided to the NN is possible. Thereby, larger datasets can be processed. Here, intermediate results obtained for one patch in a given iteration of the segmentation process can be propagated to another patch prior to processing this other patch in the given iteration. This reduces the run time of the segmentation process. For training of the NN, a click generation protocol can be used to synthesize one or more localized user inputs that correct or ascertain an a-priori estimate of the segmentation. Thus, the user feedback can be automatically synthesized. This reduces the annotation effort for the training and thus generally yields better training results. The click generation protocol can be based on a distance transformation of a difference between a ground-truth segmentation and the a-priori estimate of the segmentation. Thereby, the human annotation process can be mimicked. A better training result can be obtained, e.g., if compared to scenarios where clicks are randomly placed.

User inputs that ascertain or correct a current estimate of the segmentation are encoded to be input to the NN. An encoding of user inputs that ascertain or correct a current estimate of the segmentation can be determined based on a distance transformation operating based on these localized user inputs and represented in a 3D array data structure. Distance transformations are also labelled distance map or distance field. Such map labels each pixel or voxel based on it distance to an obstacle pixel or voxel, here the location or locations of user inputs. By such encoding, a more accurate feedback can be provided to the NN. Better results for the segmentation are obtained.

The disclosed techniques allow for an interactive, model-based segmentation in three dimensions, while prior art references only allow for operating in two dimensions or were not interactive or non-model based. These restrictions of the prior art limit leveraging domain knowledge for quick and swift interactive user experience; a deficiency overcome by the disclosed techniques.

According to an example, a computer-implemented method includes performing an interactive segmentation process. Thereby, a segmentation of the target volume is determined. The target volume is depicted by a volumetric imaging dataset. The interactive segmentation process includes multiple iterations. Each iteration of the interactive segmentation process includes determining, using a NN, a respective estimate of the segmentation and obtaining, from a user interface, one or more localized user inputs that correct or ascertain the respective estimate of the segmentation. The NN includes multiple inputs. These multiple inputs include the estimate of the segmentation determined in a preceding iteration of the multiple iterations, an encoding of the one or more localized user inputs obtained in the preceding iteration, as well as the volumetric imaging dataset.

The volumetric imaging dataset can be a medical volumetric imaging dataset, e.g., CT imaging dataset or an MRI dataset.

The target volume can be a ROI defined by a radiologist, e.g., a certain organ or a certain anatomy. For instance, blood vessels may be segmented. A certain organ such as the liver or the heart may be segmented. The coronary system may be investigated and segmented.

The NN can be a deep NN including multiple convolutional layers. A convolutional NN (CNN) can be employed. A U-Net architecture may be employed. See, e.g., Du, Getao, et al. "Medical image segmentation based on u-net: A review." Journal of Imaging Science and Technology 64 (2020): 1-12.

The user interface can be a graphical user interface (GUI) that implements the one or more localized user inputs such as clicks, nudges, shape definitions provided by a user via a mouse input or another input.

The multiple inputs to the NN can be 3-D array data structures. The array positions of the 3-D array data structures can correspond to a spatial grid (xyz-positions) that is predetermined, e.g. in accordance with the volumetric imaging dataset.

A computer program or a computer program product or a computer-readable storage medium includes program code. The program code can be executed by at least one processor. The at least one processor, upon loading and executing the program code, is caused to perform the computer-implemented method as outlined above.

A processing device includes a processor and a memory. The memory is configured to store program code. The processor is configured to load the program code and to execute the program code. Execution of the program code causes the at least one processor to perform the above-described computer implemented method.

A computer-implemented method of training a NN is disclosed. The NN is used for determining an a-posterior estimate of the segmentation of the target volume that is depicted by a imaging dataset, e.g., a 3-D or 2-D imaging dataset. The NN includes multiple inputs. These multiple inputs include an a-priori estimate of the segmentation, an encoding of one or more localized user inputs that correct or ascertain the a-priori estimate of the segmentation, as well as the volumetric imaging dataset. The method includes synthesizing the one or more localized user inputs based on a comparison between a ground-truth segmentation of the target volume and the a-priori estimate of the segmentation. The method also includes training the NN based on a loss that is determined based on a comparison between the ground-truth segmentation and the a-priori estimate of the segmentation. The a-posterior estimate of the segmentation is determined based on the one or more localized user inputs and the a-priori estimate of the segmentation and using the NN in its current training state.

The one or more localized user inputs can thus be synthesized using a click generation protocol.

The NN, once trained, can be used as part of an interactive segmentation process that includes multiple iterations, wherein the NN is used in each iteration to predict a current estimate of the segmentation based on a previous estimate of the segmentation. In such a scenario, the previous estimate of the segmentation corresponds to the a-priori estimate of the segmentation and the current estimate of the segmentation (predicted by the NN in a given iteration) corresponds to the a-posterior estimate of the segmentation.

A computer-implemented method includes performing an interactive segmentation processed to determine a segmentation of the target region that is depicted by an imaging dataset. The imaging dataset may be a 2D image or a 3D volumetric imaging dataset. The interactive segmentation process includes multiple iterations. Each iteration of the interactive segmentation process includes determining, using a NN, a respective estimate of the segmentation of the target region and obtaining, from a user interface, one or more localized user inputs that correct or ascertain the respective estimate of the segmentation. The NN includes multiple inputs. The multiple inputs include the estimate of the segmentation that is determined in a preceding iteration of the multiple iterations (as a-priori estimate), and an encoding of one or more localized user inputs that are obtained in a preceding iteration, as well as the volumetric imaging dataset. The encoding of the one or more localized user inputs is determined based on distances between each one of the one or more localized user inputs and grid positions of the predetermined spatial grid. Thus, the encoding is a distance transformation of a 3-D representation of the localized user inputs. A distance metric that is used for determining the distances comprises continuous output variables. For instance, a Euclidean distance metric can be used.

It is to be understood that the features mentioned above and those yet to be explained below may be used not only in the respective combinations indicated, but also in other combinations or in isolation without departing from the scope of the invention.
FIG. 1 illustrates a processing device according to various examples.
FIG. 2 is a flowchart of a method according to various examples.
FIG. 3 schematically illustrates a processing pipeline of an interactive segmentation process according to various examples.
FIG. 4 schematically illustrates patch-by-patch processing of a NN according to various examples.
FIG. 5 is a flowchart of a method of training a NN to predict a segmentation of an imaging dataset according to various examples.
FIG. 6 schematically illustrates aspects with respect to a click generation protocol according to various examples.

Some examples of the present disclosure generally provide for a plurality of circuits or other electrical devices. All references to the circuits and other electrical devices and the functionality provided by each are not intended to be limited to encompassing only what is illustrated and described herein. While particular labels may be assigned to the various circuits or other electrical devices disclosed, such labels are not intended to limit the scope of operation for the circuits and the other electrical devices. Such circuits and other electrical devices may be combined with each other and/or separated in any manner based on the particular type of electrical implementation that is desired. It is recognized that any circuit or other electrical device disclosed herein may include any number of microcontrollers, a graphics processor unit (GPU), integrated circuits, memory devices (e.g., FLASH, random access memory (RAM), read only memory (ROM), electrically programmable read only memory (EPROM), electrically erasable programmable read only memory (EEPROM), or other suitable variants thereof), and software which co-act with one another to perform operation(s) disclosed herein. In addition, any one or more of the electrical devices may be configured to execute a program code that is embodied in a non-transitory computer readable medium programmed to perform any number of the functions as disclosed.

In the following, embodiments of the invention will be described in detail with reference to the accompanying drawings. It is to be understood that the following description of embodiments is not to be taken in a limiting sense. The scope of the invention is not intended to be limited by the embodiments described hereinafter or by the drawings, which are taken to be illustrative only.

The drawings are to be regarded as being schematic representations and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling. A coupling between components may also be established over a wireless connection. Functional blocks may be implemented in hardware, firmware, software, or a combination thereof.

Various examples of the disclosure pertain to segmentation of medical imaging data. Specifically, various examples pertain to segmentation of volumetric imaging datasets, i.e., 3-D imaging datasets. Medical volumetric imaging datasets can be segmented.

Volumetric imaging data can depict a certain volume within the body. Volumetric imaging data can be obtained using one or more volumetric imaging techniques such as Computed Tomography, Magnetic Resonance Tomography, ultrasound imaging, positron emission tomography. While the particular body part that is depicted by the volumetric imaging dataset is not germane for the techniques disclosed herein, it would be possible to detect a cardiovascular system, using CTA.

Volumetric imaging datasets include a 3-D array of voxels. They can be rendered using various techniques, e.g., depicting 2-D slices.

Various techniques employ deep-learning-based segmentation processes. Here, a NN, e.g., a CNN, is used as part of the segmentation process to enable the segmentation. For instance, the NN can output a segmentation mask. The segmentation mask distinguishes between regions being part of the segmentation and regions being outside of the segmentation. Accordingly, it would be possible that each voxel is assigned to either true (i.e., being part of the segmentation) or false (i.e., being not part of the segmentation).

Various techniques are based on the finding that for fully automated "Deep Learning"-based segmentation processes, there exists a lack of control over the generated solutions. This is because of to the lack of interaction and the inherent inability of the segmentation process to consider user feedback. Thus, fully-automated segmentation processes provide only suboptimal solutions, resulting from limited data during training or the use of noisy labels. The manual correction of volumetric segmentations, however, as needed for the clinical assessment, is highly tedious and time-consuming and hence hinders the clinical workflow.

Additionally, fully-automatic segmentation processes tend to be domain specific, i.e., cannot be easily transferred from one body region to another body region without loss of accuracy. This limits their applicability to various specific subtasks. This limits the practical use of such fully-automatic segmentation processes in the clinical routine.

The disclosed techniques mitigate or overcome such limitations of other segmentation processes.

According to examples, an interactive segmentation process is disclosed. The interactive segmentation process includes multiple iterations. At each iteration, a current estimate of the segmentation is determined using a NN; then, user feedback is gathered and based on this user feedback, in the next iteration, the estimate of the segmentation is refined.

The techniques disclosed herein allow for a guided correction of the estimate of the segmentation and thus reducing the number of needed interactions for high-quality annotations. The disclosed techniques enable a general-purpose solution for interactive annotation of organs and tissues within arbitrary body regions.

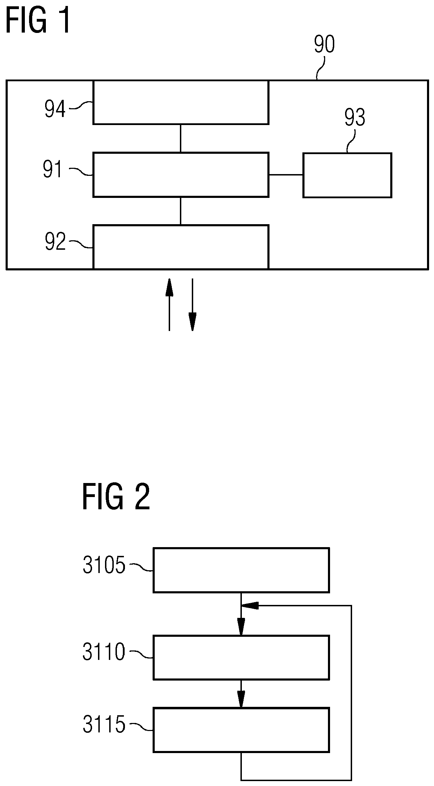

FIG. 1 schematically illustrates a processing device 90 according to various examples. The processing device 90 includes a processor 91 and the memory 93. The processor 91 can load program code from the memory 93 and execute the program code. The processor 91 can load volumetric medical imaging datasets via a communication interface 92, e.g., from a picture archiving system or another database. The processor 91 can provide results of a segmentation process via the communication interface 92, e.g., as metadata associated with volumetric imaging datasets. The processor can interact with a user via a human machine interface (HMI) 94, e.g., providing a GUI. The processor, upon loading and executing program code, can perform techniques as disclosed herein, e.g., training a NN used as part of an interactive segmentation process; performing the interactive segmentation process; re-training the NN. Further, one or more aspects of FIG. 2 can be executed by the processor upon loading the program code.

FIG. 2 is a flowchart of a method according to various examples. One or more of the boxes of FIG. 2 can be executed by the computing device 90 of FIG. 1.

At box 3105, a NN of a deep-learning-enabled interactive-segmentation process is trained. This is based on a ground-truth for the segmentation.

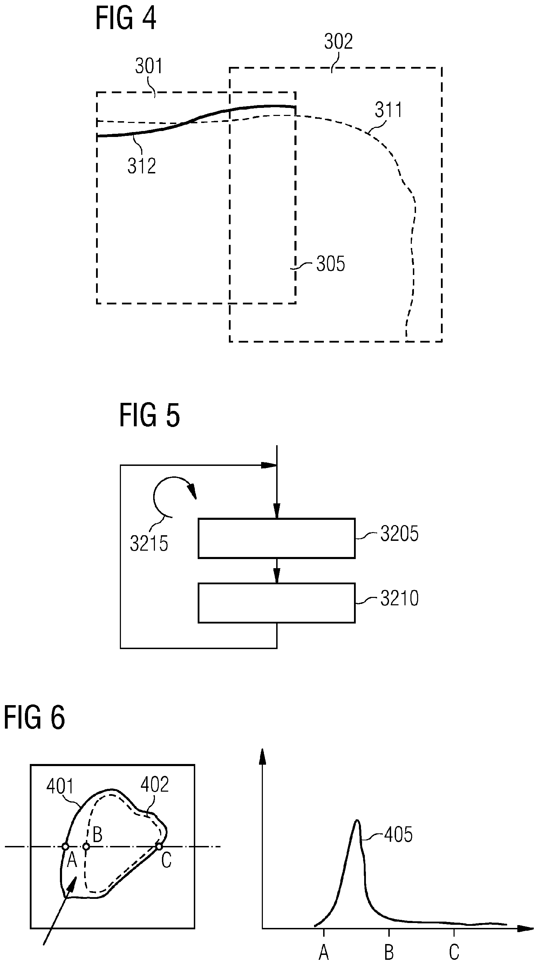

A possible implementation of box 3105 will be explained in connection with FIG. 5.

As part of box 3105, a click generation protocol can be used to synthesize localized user inputs that affirm or correct current estimates of the segmentation. This helps to mimic the iterative and interactive process. Details with respect to the click generation protocol will be explained later in connection with FIG. 6.

Once the NN has been trained, the method commences at box 3110.

At box 3110, inference tasks are solved by the interactive segmentation process. I.e., a segmentation of a target volume depicted by a volumetric imaging dataset is determined, without available ground truth.

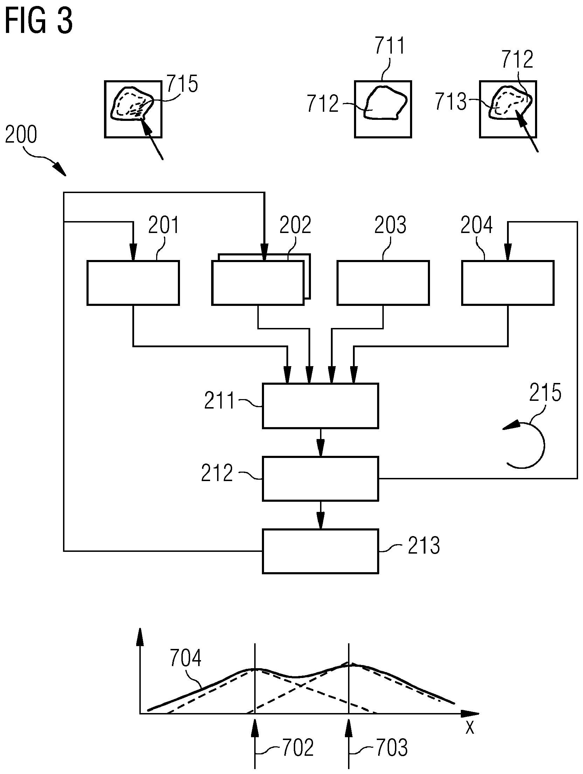

The interactive segmentation process of box 3110 includes multiple iterations (details will be explained later with respect to FIG. 3). For each iteration, one or more localized user inputs are obtained. These one or more localized user inputs provide feedback regarding the current estimate of the segmentation obtained in that iteration. The one or more localized user inputs can affirm or correct the current estimate of the segmentation.

It is optional to re-train the NN at box 3115, based on a result of an interactive segmentation process executed at box 3110 (see feedback loop in FIG. 2). I.e., the final segmentation can be used as ground truth for the re-training at box 3115.

The re-training of box 3115 can employ techniques of continued learning. The continued learning can build upon existing weights of the NN from the training of box 3105; i.e., these weights are refined (rather than discarded). For instance, it would be possible to retain a fraction of the initial training data used at box 3105 and consider this fraction also in the re-training at box 3115. Other techniques are known to the skilled person and can be employed.

The continued learning process employed at box 3115 can use the encodings of the localized user inputs obtained during the interactive segmentation process of box 3110 as inputs to the NN. In other words, the history of localized user inputs may be retained during box 3110 and then provided to the continued learning process executed at box 3115. Thereby, multiple iterations of the interactive segmentation process can be resembled in the continued learning process of box 3115, each iteration being associated with the respective part of the history of localized user inputs obtained from box 3110. A click generation protocol is then not required, because the actual user inputs are available.

FIG. 3 schematically illustrates aspects with respect to the interactive segmentation process 200. FIG. 3 illustrates a processing pipeline of the interactive segmentation process 200.

The interactive segmentation process 200 includes multiple iterations 215. For each iteration 215, the NN 211 predicts a current estimate 212 of the segmentation (a-posterior estimate). At the final iteration 215, this current estimate is used as the final result.

For each iteration 215, one or more localized user inputs 213 are obtained correcting or ascertaining the respective estimate of the segmentation. The user inputs 213 are input to the NN 211 in the subsequent iteration 215. By means of the localized user inputs 213, the user can provide feedback regarding the current estimate 212 of the segmentation. For instance, the user can confirm that the current estimate 212 of the segmentation correctly delimits the ROI against surrounding regions in a certain area. This is sometimes referred to as a positive user interaction. The user may also indicate, via the user interface, that the current estimate 212 of the segmentation in a certain area wrongly assumes that a surrounding region is part of the ROI, or vice versa. This is sometimes referred to as a negative user action. There are various options available for implementing the user interface for obtaining the one or more localized user inputs that correct or ascertain the respective estimate of the segmentation. For instance, 2-D slices of the volumetric imaging dataset could be presented to the user with a respective indication of the corresponding part of the current estimate of the segmentation. Then, the user could provide clicks that either ascertain or correct the respective estimate of the segmentation. Beyond such 0-D clicks, e.g., using a pointer device such a mouse, other scenarios are possible, e.g., 1-D contouring or 1-D or 2-D nudges. The user may also simply indicate that the current estimate of the segmentation in the respective region is less accurate than the prior estimate of the segmentation. The user may provide input regarding the shape of the boundary region delimiting region of interest from surrounding regions.

FIG. 3 illustrates the inputs 201-204 provided to the NN 211.

Each input 201-204 can have one or more channels.

In the scenario of FIG. 3, the NN 211 operates in 3-D. Because the NN 211 operates in 3-D, the inputs 201-204 are 3-D array data structures. Each 3-D array data structure that includes values for a predetermined grid positions of a 3-D spatial grid. For instance, the 3-D array data structure for the volumetric imaging dataset (input 203) can include voxels that encode respective contrast values at respective spatial positions.

The inputs 201-204 are, in detail:
Input 203: the volumetric imaging dataset 711. Illustrated in FIG. 3 is the 3-D volumetric imaging dataset 711 (for sake of simplicity, a 2-D representation is shown) including the target volume 712 forming a region of interest to be segmented.
Input 204: the estimate 713 of the segmentation of the preceding iteration 215.
Input 201: the encoding 715 of one or more localized user inputs 213 obtained in the preceding iteration. It would be possible to determine respective values for ascertaining user inputs and separately for corrective user inputs, to thereby yield two respective channels (corrective user input: channel A; affirmative user input: channel B) of the encoding forming the input 201 (and the input 202). The encoding 715 shows maximum values in a region where the previous estimate 713 of the segmentation deviates from the actual target volume 712 (marked by an arrow in FIG. 3), because corrective user inputs would be placed there.

Optional input 202: the encodings of one or more localized user inputs obtained for one or more further preceding iterations, i.e., prior to the preceding iteration 215.

In other words, the encoding of input 201 can pertain to the preceding iteration 215 and the encodings of input 202 could pertain to one or more iterations even prior to that preceding iteration 215 (the number of channels of the input 202 depends on the count of the one or more iterations for which the user input history is considered).

Similar types of encodings can be used for the one or more user inputs of the previous iteration 215 and the one or more preceding iterations 215.

By means of the input 202, an extended click history can be considered. In contrast to previous methods, the proposed system further explicitly considers the history of previous correction steps, being subject to the above-mentioned embedding methodology. This comes along with a variety of benefits: By considering the input 202, it is possible to condition the NN 211 on avoiding the overwriting of previous correction steps. As a result of this, previous user interactions are consistently considered, and in contrast to previous interactive segmentation methods cannot be overridden until a manual reset of the modification history. Implementing the history as part of the system's optimization target allows the systems to explicitly model the sequence of corrections as part of the correction cycle itself. Resulting from this, the system's correction proposals adapt to the user interaction. As a result of this, the system can automatically detect whether it tends to move segmentation boundaries too far inside or outside of the desired region - a scenario which is particularly important for tissues with changing image intensity, such as is the case for contrast-enhanced tissue, and as it may result from the choice of the scanning protocol. In these cases, a single click can guide the system towards choosing more restrictive or more tolerant segmentations, thus adapting to the user's preferred boundary definition.

As a general rule, various kinds and types of encodings 715 may be used for the localized user inputs (inputs 201, 202 to the NN 211). The type of encoding 715 ensures that the NN 211 can correctly understand and interpret the localized user inputs. In one example, a binary sphere encoding may be used. Here, at the position of a click provided by the user, a sphere having a predetermined size is located. The respective 3-D array data structure implementing the encoding has values of, e.g., "1" within the sphere; and "0" outside of the sphere, for an ascertaining click. Thus, a binary distance metric is used that abruptly (non-continuously) changes from one value to another value. The 3-D array data structure has values of, e.g., "-1" within the sphere and "0" outside the sphere for a corrective click. While such approach is generally possible, it comes with a variety of disadvantages: The sphere sizes are a free hyperparameter that must be subject to optimization. As different sizes are expected to perform well for different scenarios, this includes a per-case modification. Using spheres limits the system's capability of border modifications, as correctable errors need to have at least the size of the disks themselves. Sphere representations result in a sparse representation of clicks. Notably, by this definition, the encodings sparsity increases exponentially with increasing dimensionality and is thus already infeasible for three dimensions, as well as any further.

In a further example, the encoding 715 of the one or more user inputs is determined based on distances between each one of the one or more localized user inputs and grid positions of a predetermined 3-D spatial grid. A distance metric used for determining the distances includes continuous output variables. The 3-D spatial grid can be determined by the 3D array data structure that is input to the NN 211. For instance, the Euclidean distance metric may be used. The 3-D array data structure implementing the encodings of inputs 201, 202 represents the click position in the continuous space through per-voxel distances to the positions of correction. For such distance encoding 715, all aforementioned limitations of the binary sphere encoding are solved, by a) the encoding being hyperparameter-free, by b) introducing no limitations of minimal edits (i.e., user inputs can be localized in-between grid positions of the 3-D grid) and c) by avoiding sparsity through using a dense representation. Further, it allows for the representation of multiple clicks using a single input. This means that the values of the 3-D array data structure implementing the encodings of inputs 201, 202 is determined by a superposition of multiple partial values, each partial value being dependent on the distance to a certain click.

Such example of a continuous distance metric is illustrated in FIG. 3. Illustrated are Euclidean distance values 704 (dashed lines) obtained from two localized user clicks 702, 703 (as examples of the localized user inputs 213) that both correct the current estimate of the segmentation (the distance is represented in 1-D for sake of simplicity; but Euclidean distances or other distance metrics are likewise available in 3-D). The distance values 704 (full line) are obtained from a superposition of respective functions that, based on the Euclidean distance, linearly take smaller values for larger distances to the position of the localized user inputs 702, 703. The distance values 704 are then represented by the encoding 715 provided as the inputs 201, 202.

The distance values 704 are determined by a superposition of distances to multiple localized user inputs. Due to such superposition, a normalization of the values of the 3-D array data structure can be used. This can be achieved by pre-processing using convolutional layers of the NN 211, allowing the normalization itself to be part of the systems self-optimization process during the training (cf. box 3105 in FIG. 2). Beyond such normalizing that is implemented by one or more layers of the NN 211, the normalization could also be implemented as part of a separate pre-processing algorithm upstream of the NN 211.

Situations can occur where the 3-D array data structures implementing the inputs 201-204 have a dimensionality that is too large to process in one patch by the NN 211. This can be the case for volumetric imaging datasets that have a high resolution and/or a large field of view. In such scenarios, patch-by-patch processing can be employed. Here, the 3-D array data structures implementing the inputs 201-204 can be subdivided into multiple patches (each having a respectively smaller size). Individual patches can then be separately processed by the NN 211, e.g., multiplexed in time. Thus, within each iteration 215, multiple patch sub-loops can be formed.

For patch-by-patch processing, a spatial grid used for determining the encoding of the localized user inputs can extend across multiple patches. This means that the distance metric used for encoding user inputs is calculated across patches. For instance, if a user input is located in a region associated with a first patch, the encoding of user inputs will also have distance values affected by that user input in a region associated with a second patch. I.e., simply speaking, click distances are tracked across patch boundaries. Multiple patches of the encoding of the user inputs are determined based on distances between the 3-D grid positions and the localized user inputs that are larger than the patch size, i.e., span beyond patch boundaries.

According to examples, a patch-by-patch processing in each iteration 215 can employ an iterative sub-volume propagation, which ensures a flow of information from an updated patch to all other patches in the remaining volume. This means that a part of a first patch of the estimate of the segmentation determined in a given iteration 251 is used to refine an overlapping part of a second patch of the estimate of the segmentation determined in the preceding iteration, prior to determining the second patch of the estimate of the segmentation in the given iteration.

This means that a result of one patch can be propagated to another patch even within a given iteration 215. The prior estimate of the another patch is updated based on the estimate of the segmentation in the patch already processed. This is possible where there is an overlap between the patches.

Such propagation of the estimate of the segmentation determined in a given iteration for a given patch to another patch can be implemented by a replacement of the prior estimate of the segmentation in the another patch by the respective current estimate of the segmentation obtained for the given patch. Such replacement can create discontinuities at the borders of the overlap region, where the previous estimate of the segmentation and the current estimate of the segmentation meet. To avoid this, it would be possible to use interpolation, e.g., via kernel density estimation, shape interpolation, etc. This ensures smooth transitions between the current estimate of the segmentation determined for the given patch in the previous estimate of the segmentation determined for the another patch at the border of the overlap region between those two patches. This is also illustrated in FIG. 4.

FIG. 4 schematically illustrates a patch 301 and another patch 302 (here, a 2-D representation of the patches is used for sake of simplicity; but the patches 301, 302 can generally have 3-D extension). Illustrated is the estimate 311 of the segmentation of the preceding iteration 215. Then, by the patch-by-patch processing the current estimate 312 of the segmentation is determined for the patch 301. This current estimate 312 deviates from the previous estimate 311. The current estimate 312 is used to update the previous estimate 311 in the overlap region 305 of the patches 301, 302, prior to processing, in the same iteration 251, the patch 302.

As a general rule, propagation of the current estimate of the segmentation determined in a given patch to one or more further patches, prior to processing the one or more further patches in the NN, can be limited to a local neighborhood of the given patch. The local neighborhood can be defined by prior knowledge of anatomical features. For instance, for blood vessels as a target volume to be segmented, all patches along a centerline of the respective blood vessels are updated; while such patches offset from the centerline need not to be updated. I.e., in other words, the refining of the prior estimate of the segmentation can be restricted based on prior knowledge regarding an extension of the target volume. This can increase runtime efficiency.

FIG. 5 is a flowchart of a method according to various examples. The method of FIG. 5 pertains to training a NN that is used for determining an a-posterior estimate of a segmentation of a target volume depicted by a volumetric imaging dataset. The NN can include multiple inputs that include an a-priori estimate of the segmentation. The inputs can further include an encoding of one or more localized user inputs correcting or ascertaining the a-priori estimate of the segmentation, as well as the volumetric imaging dataset.

For instance, the NN trained using the method of FIG. 5 can be used to determine current estimates in an iterative segmentation process such as the iterative segmentation process 200 illustrated in FIG. 3. The method of FIG. 5 can be used to train the NN 211.

The method of FIG. 5 can be executed by a processing device, e.g., the processing device 90. More specifically, the method of FIG. 15 can be executed by the processor 91 upon loading program code from the memory 93 and upon executing the program code.

The method of FIG. 5 could be part of box 3105 of the method of FIG. 2.

At box 3205, one or more localized user inputs are synthesized based on a comparison between a ground-truth segmentation of the target volume and the a-priori estimate of the segmentation. The a-priori estimate is an input to the NN. The ground-truth segmentation can be obtained from manual user annotation or from alternative measurement techniques. The ground-truth segmentation can be predefined.

The synthesizing of the one or more localized user inputs can be implemented by a so-called "click generation protocol".

Such synthesizing of one or more localized user inputs mimics human behavior for providing localized user inputs that would correct or ascertain the a-priori estimate of the segmentation. By synthesizing the one or more localized user inputs, the respective input to the NN during the training of the NN is automatically generated. This can reduce the amount of expert time; and thus leads to a more accurate training.

Once the one or more localized user inputs have been synthesized at box 3205, the method commences at box 3210. Here, the NN is trained based on a loss. The loss is determined based on a comparison between the ground-truth segmentation and the a-posterior estimate of the segmentation that is determined based on the one or more localized user inputs (that have been synthesized) and the a-priori estimate of the segmentation, using the NN in its current training state.

Conventional training techniques could be used, e.g., back-propagation implementing a gradient descent optimization of the weights of the NN.

As illustrated in FIG. 5, this could be repeated. Specifically, it would be possible that for each iteration 3215, different a-priori estimates of the segmentation are presented to the click generation protocol to synthesize respective localized user inputs.

It would be possible to consider a click history, as explained above in connection with FIG. 3: input 202.

Next, in connection with FIG. 6, details of the synthesizing of user inputs are described (cf. FIG. 5: box 3205). FIG. 6, left illustrates the ground-truth segmentation 401 and the a-priori estimate 402 of the segmentation (illustrated is a 2D representation for sake of simplicity). As illustrated in FIG. 6, there is a certain region where the a-priori estimate 402 significantly deviates from the ground-truth 401. This area is marked by an arrow in FIG. 6.

Then, a difference between the ground-truth 401 segmentation and the a-priori estimate 402 of the segmentation can be determined and a distance transformation can be applied to this difference of the ground-truth 401 and the a-priori estimate 402 of the segmentation. Based on the output of the distance transformation, a spatial probability density 405 for the presence of a corrective localized user input is determined. As illustrated in FIG. 6, the spatial probability density for presence of a corrective localized user input maximizes about halfway in between points A and B along the dashed line in FIG. 6. This mimics the human behavioral tendency to place corrections in areas of high error density, while simultaneously ensuring that clicks might be placed at every potential location of an error between the ground-truth 401 and the a-priori estimate 402.

Summarizing, at least the following EXAMPLES have been disclosed:

### EXAMPLES

EXAMPLE 1. A computer-implemented method, comprising:
- performing an interactive segmentation process (200) to determine a segmentation of a target volume (712) depicted by a volumetric imaging dataset (203, 711), the interactive segmentation process (200) comprising multiple iterations (215), each iteration (215) of the interactive segmentation process (200) comprising determining, using a neural network algorithm (211), a respective estimate (212) of the segmentation and obtaining, from a user interface, one or more localized user inputs (213, 702, 703) correcting or ascertaining the respective estimate (212) of the segmentation,

wherein the neural network algorithm (211) comprises multiple inputs (201, 202, 203, 204),
wherein the multiple inputs (201, 202, 203, 204) comprise the estimate (212) of the segmentation determined in a preceding iteration (215) of the multiple iterations (215), an encoding (715) of the one or more localized user inputs (213, 702, 703) obtained in the preceding iteration (215), and the volumetric imaging dataset (203, 711).

EXAMPLE 2. The computer-implemented method of EXAMPLE 1,
wherein the encoding (715) of the one or more localized user inputs (201, 202, 203, 204) is determined based on distance values (704) between each one of the one or more localized user inputs (213, 702, 703) and grid positions of a predetermined three-dimensional spatial grid,
wherein a distance metric used for determining the distance values (704) comprises continuous output variables.

EXAMPLE 3. The computer-implemented method of EXAMPLE 2,
wherein the distance metric is a Euclidean distance metric.

EXAMPLE 4. The computer-implemented method of EXAMPLE 2 or 3, further comprising:
- normalizing the distance values (704) between each one of the one or more localized user inputs (213, 702, 703) and the grid positions across the predetermined three-dimensional spatial grid.

EXAMPLE 5. The computer-implemented method of EXAMPLE 4,
wherein said normalizing is applied by one or more layers of the neural network algorithm (211).

EXAMPLE 6. The computer-implemented method of any one of EXAMPLEs 2 to 5,
wherein each estimate of the segmentation is determined by multiplexed processing, using the neural network algorithm (211), of multiple patches (301, 302) determined for each of the multiple inputs (201, 202, 203, 204),
wherein the predetermined three-dimensional spatial grid used for determining the encoding (715) of the one or more localized user inputs (201, 202, 203, 204) globally extends across the multiple patches (301, 302).

EXAMPLE 7. The computer-implemented method of EXAMPLE 6,
wherein the one or more localized user inputs (213, 702, 703) are obtained, from the user interface, for a region corresponding to a subset of the multiple patches,
wherein the method further comprises:
   - determining the multiple patches of the encoding (715) of the one or more localized user inputs (213, 702, 703) based on the distances spanning beyond the subset of the multiple patches (301, 302).

EXAMPLE 8. The computer-implemented method of any one of EXAMPLEs 2 to 7,
wherein at least one of the one or more localized user inputs (213, 702, 703) is arranged in-between adjacent grid positions of the predetermined three-dimensional spatial grid.

EXAMPLE 9. The computer-implemented method of any one of the preceding EXAMPLEs,
wherein each estimate (212) of the segmentation is determined by multiplexed processing, using the neural network algorithm (211), of multiple patches determined for each of the multiple inputs (201, 202, 203, 204), the multiple patches being spatially overlapping,
wherein a part of a first patch of the estimate (212) of the segmentation determined in a given iteration (215) is used to refine an overlapping part of a second patch of the estimate (212) of the segmentation determined in the preceding iteration (215), prior to determining the second patch of the estimate (212) of the segmentation in the given iteration (215) .

EXAMPLE 10. The computer-implemented method of EXAMPLE 9,
wherein said refining is restricted based on prior knowledge regarding a spatial extension of the target volume.

EXAMPLE 11. The computer-implemented method of any one of the preceding EXAMPLEs,
wherein the multiple inputs (201, 202, 203, 204) of the neural network algorithm (211) further comprise: the encodings (715) of the one or more localized user inputs (213, 702, 703) obtained in one or more further preceding iterations (215) prior to the preceding iteration (215).

EXAMPLE 12. The computer-implemented method of any one of the preceding EXAMPLEs,
wherein each one of the multiple inputs (201, 202, 203, 204) is represented by at least one respective three-dimensional array data structure that includes values for predetermined grid positions of a three-dimensional spatial grid.

EXAMPLE 13. The computer-implemented method of any one of the preceding EXAMPLEs, further comprising:
- upon completion of the interactive segmentation process, applying (3115) a continued learning process to re-train the neural network algorithm (211), the continued training process using the segmentation of the target volume determined using the interactive segmentation process as ground truth.

EXAMPLE 14. The computer-implemented method of EXAMPLE 13,
wherein the continued learning process uses the encodings of the one or more localized user inputs obtained during interactive segmentation process as inputs to the neural network algorithm.

EXAMPLE 15. A computer-implemented method of a training a neural network algorithm (211) used for determining an a-posterior estimate (212) of a segmentation (713) of a target volume (712) depicted by an imaging dataset, the neural network algorithm (211) comprising multiple inputs (201, 202, 203, 204), the multiple inputs (201, 202, 203, 204) comprising an a-priori estimate (204, 402) of the segmentation (713), an encoding (715) of one or more localized user inputs (213, 702, 703) correcting or ascertaining the a-priori estimate (204, 402) of the segmentation (713), and the imaging dataset (203),
wherein the method comprises:
- synthesizing (3205) the one or more localized user inputs (213, 702, 703) based a comparison between a ground-truth segmentation of the target volume and the a-priori estimate of the segmentation, and
- training (3210) the neural network algorithm (211) based on a comparison between the ground-truth segmentation (401) and the a-posterior estimate (204, 402) of the segmentation, the a-posterior estimate (204, 402) of the segmentation being determined based on the one or more localized user inputs and the a-priori estimate (204, 402) of the segmentation and using the neural network algorithm in its current training state.

EXAMPLE 16. The computer-implemented method of EXAMPLE 15,
wherein the comparison comprises a distance transformation of a difference between the ground-truth segmentation (401) and the a-priori estimate (204, 402) of the segmentation.

EXAMPLE 17. The computer-implemented method of EXAMPLE 16,
wherein a spatial probability density (405) for a presence of a localized user input is determined based on an output of the distance transformation.

EXAMPLE 18. A computer-implemented method, comprising:
- performing an interactive segmentation process to determine a segmentation of a target region depicted by an imaging dataset, the interactive segmentation process comprising multiple iterations, each iteration of the interactive segmentation process comprising determining, using a neural network algorithm, a respective estimate of the segmentation of the target region and obtaining, from a user interface, one or more localized user inputs correcting or ascertaining the respective estimate of the segmentation,

wherein the neural network algorithm comprises multiple inputs,
wherein the multiple inputs comprise the estimate (212) of the segmentation determined in a preceding iteration of the multiple iterations, an encoding (715) of the one or more localized user inputs obtained in the preceding iteration, and the imaging dataset,
wherein the encoding (715) of the one or more localized user inputs is determined based on distances between each one of the one or more localized user inputs and grid positions of a predetermined spatial grid,
wherein a distance metric used for determining the distances comprises continuous output variables.

EXAMPLE 19. A computer program comprising program code executable by at least one processor, the at least one processor, upon executing the program code, performing the method of any one of the preceding EXAMPLEs.

Although the invention has been shown and described with respect to certain preferred embodiments, equivalents and modifications will occur to others skilled in the art upon the reading and understanding of the specification. The present invention includes all such equivalents and modifications and is limited only by the scope of the appended claims.

For instance, various examples have been disclosed in connection with a NN that can process 3-D array data structures, e.g., that operates directly based on a volumetric imaging dataset. However, as a general rule, some of the techniques disclosed herein can also be applied to NNs that operate in 2-D, e.g., for interactive segmentation processes used for segmenting 2-D image data. For instance, the click generation protocol that operates based on a distance transformation applied to a difference between the ground-truth segmentation and the a-priori estimate of the segmentation can be equally applied in 2-D. Similarly, encodings of ascertaining or corrective localized user inputs associated with an a-priori estimate of the segmentation can be equally applied to 2-D images and 2-D segmentations.

For further illustration, while various examples have been disclosed in the context of the segmentation of medical imaging datasets, similar techniques may also be applied to other imaging datasets, e.g., aerial images, industrial workpiece imaging, etc.

## Claims

1. A computer-implemented method, comprising:
- performing an interactive segmentation process (200) to determine a segmentation of a target volume (712) depicted by a volumetric imaging dataset (203, 711), the interactive segmentation process (200) comprising multiple iterations (215), each iteration (215) of the interactive segmentation process (200) comprising determining, using a neural network algorithm (211), a respective estimate (212) of the segmentation and obtaining, from a user interface, one or more localized user inputs (213, 702, 703) correcting or ascertaining the respective estimate (212) of the segmentation,
wherein the neural network algorithm (211) comprises multiple inputs (201, 202, 203, 204),
wherein the multiple inputs (201, 202, 203, 204) comprise the estimate (212) of the segmentation determined in a preceding iteration (215) of the multiple iterations (215), an encoding (715) of the one or more localized user inputs (213, 702, 703) obtained in the preceding iteration (215), and the volumetric imaging dataset (203, 711).

2. The computer-implemented method of claim 1,
wherein the encoding (715) of the one or more localized user inputs (201, 202, 203, 204) is determined based on distance values (704) between each one of the one or more localized user inputs (213, 702, 703) and grid positions of a predetermined three-dimensional spatial grid,
wherein a distance metric used for determining the distance values (704) comprises continuous output variables.

3. The computer-implemented method of claim 2, further comprising:
- normalizing the distance values (704) between each one of the one or more localized user inputs (213, 702, 703) and the grid positions across the predetermined three-dimensional spatial grid.

4. The computer-implemented method of claim 3,
wherein said normalizing is applied by one or more layers of the neural network algorithm (211).

5. The computer-implemented method of any one of claims 2 to 4,
wherein each estimate of the segmentation is determined by multiplexed processing, using the neural network algorithm (211), of multiple patches (301, 302) determined for each of the multiple inputs (201, 202, 203, 204),
wherein the predetermined three-dimensional spatial grid used for determining the encoding (715) of the one or more localized user inputs (201, 202, 203, 204) globally extends across the multiple patches (301, 302).

6. The computer-implemented method of claim 5,
wherein the one or more localized user inputs (213, 702, 703) are obtained, from the user interface, for a region corresponding to a subset of the multiple patches,
wherein the method further comprises:
- determining the multiple patches of the encoding (715) of the one or more localized user inputs (213, 702, 703) based on the distances spanning beyond the subset of the multiple patches (301, 302).

7. The computer-implemented method of any one of claims 2 to 6,
wherein at least one of the one or more localized user inputs (213, 702, 703) is arranged in-between adjacent grid positions of the predetermined three-dimensional spatial grid.

8. The computer-implemented method of any one of the preceding claims,
wherein each estimate (212) of the segmentation is determined by multiplexed processing, using the neural network algorithm (211), of multiple patches determined for each of the multiple inputs (201, 202, 203, 204), the multiple patches being spatially overlapping,
wherein a part of a first patch of the estimate (212) of the segmentation determined in a given iteration (215) is used to refine an overlapping part of a second patch of the estimate (212) of the segmentation determined in the preceding iteration (215), prior to determining the second patch of the estimate (212) of the segmentation in the given iteration (215) .

9. The computer-implemented method of any one of the preceding claims,
wherein the multiple inputs (201, 202, 203, 204) of the neural network algorithm (211) further comprise: the encodings (715) of the one or more localized user inputs (213, 702, 703) obtained in one or more further preceding iterations (215) prior to the preceding iteration (215).

10. The computer-implemented method of any one of the preceding claims,
wherein each one of the multiple inputs (201, 202, 203, 204) is represented by at least one respective three-dimensional array data structure that includes values for predetermined grid positions of a three-dimensional spatial grid.

11. The computer-implemented method of any one of the preceding claims, further comprising:
- upon completion of the interactive segmentation process, applying (3115) a continued learning process to re-train the neural network algorithm (211), the continued training process using the segmentation of the target volume determined using the interactive segmentation process as ground truth.

12. The computer-implemented method of claim 11,
wherein the continued learning process uses the encodings of the one or more localized user inputs obtained during interactive segmentation process as inputs to the neural network algorithm.

13. A computer-implemented method of a training a neural network algorithm (211) used for determining an a-posterior estimate (212) of a segmentation (713) of a target volume (712) depicted by an imaging dataset, the neural network algorithm (211) comprising multiple inputs (201, 202, 203, 204), the multiple inputs (201, 202, 203, 204) comprising an a-priori estimate (204, 402) of the segmentation (713), an encoding (715) of one or more localized user inputs (213, 702, 703) correcting or ascertaining the a-priori estimate (204, 402) of the segmentation (713), and the imaging dataset (203),
wherein the method comprises:
- synthesizing (3205) the one or more localized user inputs (213, 702, 703) based a comparison between a ground-truth segmentation of the target volume and the a-priori estimate of the segmentation, and
- training (3210) the neural network algorithm (211) based on a comparison between the ground-truth segmentation (401) and the a-posterior estimate (204, 402) of the segmentation, the a-posterior estimate (204, 402) of the segmentation being determined based on the one or more localized user inputs and the a-priori estimate (204, 402) of the segmentation and using the neural network algorithm in its current training state.

14. The computer-implemented method of claim 13,
wherein the comparison comprises a distance transformation of a difference between the ground-truth segmentation (401) and the a-priori estimate (204, 402) of the segmentation.

15. The computer-implemented method of claim 14,
wherein a spatial probability density (405) for a presence of a localized user input is determined based on an output of the distance transformation.
